(19) Europäisches Patentamt
European Patent Office
Office européen des brevets

(11) **EP 2 599 485 B1**

(12) **EUROPEAN PATENT SPECIFICATION**

(45) Date of publication and mention
of the grant of the patent:
**20.02.2019 Bulletin 2019/08**

(51) Int Cl.:
*A61K 31/232* (2006.01)     *A61K 47/10* (2017.01)
*A61K 47/34* (2017.01)     *A23D 9/00* (2006.01)

(21) Application number: **11812492.4**

(22) Date of filing: **26.07.2011**

(86) International application number:
**PCT/JP2011/066992**

(87) International publication number:
**WO 2012/014903 (02.02.2012 Gazette 2012/05)**

(54) **FAT-CONTAINING COMPOSITION AND ORAL FORMULATION CONTAINING SAME**

FETTHALTIGE ZUSAMMENSETZUNG UND ORALE FORMULIERUNG DAMIT

COMPOSITION CONTENANT DES GRAISSES ET FORMULATION ORALE LA CONTENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2011   JP 2011163147
26.07.2010   JP 2010167095**

(43) Date of publication of application:
**05.06.2013   Bulletin 2013/23**

(73) Proprietor: **FUJIFILM Corporation
Tokyo 106-8620 (JP)**

(72) Inventors:
• **HARAGUCHI, Nobuyuki**
**Ashigarakami-gun**
**Kanagawa 258-8577 (JP)**
• **SAKAGUCHI, Hiroyuki**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 659 347     WO-A2-2009/025380
JP-A- 2004 248 593     JP-A- 2010 155 799**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a fat-and-oil containing composition, more specifically, to a fat-and-oil containing composition which contains, at a high concentration, a glycerin fatty acid ester including docosahexaenoic acid as the constituent fatty acids, and an oral formulation using the fat-and-oil containing composition.

Background Art

Docosahexaenoic acid (hereinafter sometimes abbreviated to as "DHA") is an unsaturated fatty acid that is contained in fish oils in a larger amount. The bioactive capability thereof (the bioactivity on brain, eyes, etc.) has been attracting attention, and DHA and glycerin fatty acid esters including DHA as the constituent fatty acids have been used in nutritional supplements, food additives, etc.

As a technique to improve the absorbability in the body for a bioactive component hardly soluble to water such as an oil component, a technique, which is devised such that the bioactive component is contained in a composition having a self-emulsification ability and spontaneously emulsified/dispersed just by the contact of the composition with water or digestive juice, has been proposed. For example, Japanese Patent Application Laid-Open (JP-A) No. 2009-114157 discloses an emulsification composition for a soft capsule formulation having a self-emulsification ability which contains an oil component, glycerin, starch or a starch derivative and lysolecithin in order to obtain a formulation which includes a high mixing ratio of an oil component and a greatly reduced use of an emulsifier. As for a formulation that may be applied to such a self-emulsification type composition, it has been disclosed that encapsulated formulations such as soft capsules are suitable from the view point of the handleability and the ease of intake.

SUMMARY OF INVENTION

Problem to Be Solved by Invention

As a means which enables highly efficient intake of docosahexaenoic acid (DHA) while reducing the burden of the frequency and/or amount of intake, one conceivable possibility is a glycerin fatty acid ester, which contains a high proportion of DHA as the constituent fatty acids thereof, that is contained at a high concentration in a self-emulsification type fat-and-oil containing composition.

However, according to the findings of the present inventors, it became clear that if an attempt is made to obtain a self-emulsification type fat-and-oil containing composition which contains, at a high concentration, a glycerin fatty acid ester containing a high proportion of DHA as the constituent fatty acids thereof, then the oil component contained in the fat-and-oil containing composition will be separated therefrom, stability will be impaired, and the self-emulsification properties will deteriorate.

The present invention was made in view of the circumstances described above, and an object of the present invention is to provide a fat-and-oil containing composition which has favorable stability and excellent self-emulsification properties even in a case in which a glycerin fatty acid ester that contains a high proportion of docosahexaenoic acid as the constituent fatty acids thereof is contained at a high concentration, and an oral formulation using the fat-and-oil containing composition.

Means for Solving the Problem

The means for solving the problems are as follows.

[1] A fat-and-oil containing composition which comprises at least (A) an oil component, (B) glycerin, and (C) an emulsifier, the composition comprising:

a glycerin fatty acid ester, in which from 30 % by mass to 75 % by mass of constituent fatty acids are docosahexaenoic acid and is a glycerin fatty acid ester in which an average number of fatty acid esterification of hydroxy groups in the glycerin skeleton is from 2.5 to 3.0, as (A) the oil component; and
a decaglycerin fatty acid ester, in which constituent fatty acids are oleic acid, as (C) the emulsifier,
wherein respective content ratios of (A) the oil component, (B) the glycerin and (C) the emulsifier are within the following ranges relative to the total mass of (A) the oil component, (B) the glycerin and (C) the emulsifier:

(A) the oil component: from 55 % by mass to 78 % by mass,

(B) the glycerin: from 10 % by mass to 35 % by mass, and
(C) the emulsifier: from 5 % by mass to 13 % by mass, and

wherein only the specific emulsifier decaglycerin fatty acid ester, in which constituent fatty acids are oleic acid, is contained as (C) the emulsifier.

[2] An oral formulation containing the composition of [1].

Advantageous Effect of Invention

According to the present invention, it is provided a fat-and-oil containing composition which has a favorable stability and an excellent self-emulsification property even in a case in which a glycerin fatty acid ester which contains a high proportion of docosahexaenoic acid as the constituent fatty acids thereof is contained at a high concentration, and an oral formulation using the fat-and-oil containing composition..

EMBODIMENT FOR CARRYING OUT INVENTION

The fat-and-oil containing composition of the present invention is a fat-and-oil containing composition which comprises at least (A) an oil component, (B) glycerin, and (C) an emulsifier, the composition comprising:

a glycerin fatty acid ester, in which from 30 % by mass to 75 % by mass of constituent fatty acids are docosahexaenoic acid and is a glycerin fatty acid ester in which an average number of fatty acid esterification of hydroxy groups in the glycerin skeleton is from 2.5 to 3.0, as (A) the oil component; and
a decaglycerin fatty acid ester, in which constituent fatty acids are oleic acid, as (C) the emulsifier,
wherein respective content ratios of (A) the oil component, (B) the glycerin and (C) the emulsifier are within the following ranges relative to the total mass of (A) the oil component, (B) the glycerin and (C) the emulsifier:

(A) the oil component: from 55 % by mass to 78 % by mass,
(B) the glycerin: from 10 % by mass to 35 % by mass, and
(C) the emulsifier: from 5 % by mass to 13 % by mass, and

wherein only the specific emulsifier decaglycerin fatty acid ester, in which constituent fatty acids are oleic acid, is contained as (C) the emulsifier.

Hereinafter, the components of (A) the oil component, (B) the glycerin and (C) the emulsifier are each referred to as Component (A), Component (B) and Component (C), as appropriate.
In the present specification, "fat-and-oil" means an ester compound of fatty acids and glycerin (glyceride). The fat-and-oil containing composition of the present invention must contain, as the fat-and-oil, a glycerin fatty acid ester including docosahexaenoic acid.
The fat-and-oil containing composition of the present invention is a fat-and-oil containing composition which has self-emulsification properties.
The phrase "a fat-and-oil containing composition which has self-emulsification properties" as used herein means that when contacted with an aqueous liquid such as water or digestive juices, the fat-and-oil containing composition is spontaneously emulsified without any external force applied by mechanical operation.
The fat-and-oil containing composition of the present invention exhibits excellent stability and excellent self-emulsification properties by combined use, selectively and at specific amounts, of glycerin and a decaglycerin fatty acid ester in which the constituent fatty acids are oleic acid, in a case in which a glycerin fatty acid ester that includes docosahexaenoic acid as the constituent fatty acids is contained at a high concentration.
In a case in which the amount of a component in the composition is indicated in the invention, when there are plural substances corresponding to the component in the composition, the indicated amount means the total amount of the plural substances present in the composition, unless specifically stated otherwise.
In the present specification, any numerical range expressed herein using "to" refers to a range including the numerical values before and after "to" as the minimum and maximum values, respectively.
In the present specification, the term "process" encompasses an independent process, as well as a process that cannot be clearly distinguished from another process but yet achieves the expected effect of the process of interest.
The essential components and optional components contained in the composition of the present invention are described below.

[Component (A)]

The fat-and-oil containing composition of the present invention contains (A) the oil component [Component (A)], and the glycerin fatty acid ester in which from 30% by mass to 75% by mass of constituent fatty acids are docosahexaenoic acid (hereinafter sometimes referred to as "the specific glycerin fatty acid ester") is included as the oil component.

Oil components as covered by the emulsifier (C), such as the decaglycerin fatty acid ester in which the constituent fatty acids are oleic acid as described below, are not included in oil components that correspond to Component (A).

The "constituent fatty acids" in Component (A) refer to the constituent fatty acids that are included in all the glycerin fatty acid esters contained as Component (A) in the fat-and-oil containing composition of the present invention and, in the present invention, from 30% by mass to 75% by mass thereof are required to be docosahexaenoic acid.

The content ratio of docosahexaenoic acid included in Component (A) may be determined by a method for quantifying fatty acids using an alkaline decomposition method (the operations (a) to (d) as described below).

<(a) Saponification of Sample and Extraction of Fatty Acid Component>

A sample to be measured is dissolved in 1 mol/L potassium hydroxide/ethanol (containing 1% by mass of pyrogallol) solution to saponify. As an internal standard, heptadecanoic acid is used.

To the sample solution after the saponification, water, 30% by mass of sulfuric acid and diethyl ether are added. Thereafter, diethyl ether layer (organic layer) is separated therefrom and washed with water. The solvent (diethyl ether) is distilled off from the diethyl ether layer after the wash with water to obtain a fatty acid component.

<(b) Saponification>

To the fatty acid component obtained in the operation (a), 0.5 mol/L solution of sodium hydroxide in methanol is added to saponify.

<(c) Methyl Esterification>

To the sample after the saponification in the operation (b), a solution of boron trifluoride methanol complex in methanol is added to carry out methyl esterification of the fatty acid components included in the sample.

<(d) Identification of Fatty Acids by Gas Chromatography (Hydrogen Flame Ionization Detector)>

To the sample after the methyl esterification in the operation (c), hexane and saturated saline are added. Thereafter, hexane layer (organic layer) is separated therefrom. Using as a measurement sample the hexane layer after the separation, the type and amount of the fatty acids included in the sample are analyzed by gas chromatography using a hydrogen flame ionization detector.

In the specific glycerin fatty acid ester included in Component (A), the average number of fatty acid esterification of hydroxy groups in the glycerin skeleton is preferably from 2.5 to 3.0, and more preferably from 2.7 to 3.0. The average number of fatty acid esterification herein means an average value of the number of fatty acid esters per 1 molecule of the glycerin fatty acid ester which is contained as Component (A) in the fat-and-oil containing composition of the present invention.

The average number of fatty acid esterification may be calculated by an average esterification rate (%) x 3 ÷ 100.

The average esterification rate (%) is calculated from the following Formula by using the saponification value (SV), the acid value (AV) and the hydroxyl value (OHV) of the glycerin fatty acid ester, which values have been measured by "Standard Methods for Testing the Physical Properties of Fats and Oils" (established by Japan Oil Chemists' Society).

$$\text{Esterification Rate (\%)} = \{(SV - AV) \times 100\} / (OHV + SV - AV)$$

SV represents a saponification value, AV represents an acid value, and OHV represents a hydroxyl value.

The specific glycerin fatty acid ester included in Component (A) may be any of monoglyceride, diglyceride and triglyceride, and is more preferably diglyceride or triglyceride, still more preferably triglyceride.

As the specific glycerin fatty acid ester, a commercially available product may be used, and examples thereof include, for example, ALGATRIUM (manufactured by Brudy Technology), DHA-46A, DHA-70 (Maruha Nichiro Foods, inc.), etc.

As the oil component which is Component (A), other oil component other than the specific glycerin fatty acid ester may be contained. Examples of the other oil component include, for example, other fats and oils other than the specific glycerin fatty acid ester and glycerin fatty acid esters that function as Component (C); other oil components having a physical property and/or a functionality according to the purpose; and the like.

It is especially preferable that only the specific glycerin fatty acid ester be contained as Component (A) in the fat-and-oil containing composition of the present invention.

The content ratio of Component (A) in the fat-and-oil containing composition of the present invention is in a range from 55% by mass to 78% by mass relative to the total mass of Components (A), (B) and (C) in the composition, and is more preferably in a range from 60% by mass to 70% by mass.

The content ratio of Component (A) may be appropriately set within the range described above taking into consideration the content of other component used in combination.

[Component (B)]

The fat-and-oil containing composition of the present invention contains (B) the glycerin [Component (B)] in a range from 10% by mass to 35% by mass relative to the total mass of Components (A), (B) and (C) in the composition.

As the glycerin, concentrated glycerin having a concentration of 98% or more is especially preferable.

The content ratio of (B) the glycerin in the fat-and-oil containing composition of the present invention is in a range from 10% by mass to 35% by mass relative to the total mass of Components (A), (B) and (C) in the composition, and, from the viewpoints, for example, of the dispersibility and the stability of the composition, and the stability of the capsule in a case in which capsule formulation has been carried out, is preferably within a range from 15% by mass to 30% by mass.

[Component (C)]

The fat-and-oil containing composition of the present invention contains (C) the emulsifier [Component (C)], and includes, as the emulsifier, the decaglycerin fatty acid ester in which the constituent fatty acids are oleic acid (hereinafter sometimes referred to as "the specific emulsifier").

The specific emulsifier included as Component (C) is a compound which has a surface-active ability due to having, within the same molecule, both decaglycerin, which is a hydrophilic moiety, and oleic acid having an unsaturated bond, which is a hydrophobic moiety.

The specific emulsifier included as Component (C) may function as an emulsifier when the fat-and-oil containing composition of the present invention is contacted with an aqueous liquid such as water or digestive juices and exhibits self-emulsification properties.

As the specific emulsifier, a commercially available product may be used, and examples thereof include, for example, POEM J-0381V (manufactured by Riken Vitamin Co., Ltd.), SUNSOFT Q-17S (manufactured by Taiyo Kagaku Co., Ltd.), etc.

The content ratio of Component (C) in the fat-and-oil containing composition of the present invention is in a range from 5% by mass to 13% by mass relative to the total mass of Components (A), (B) and (C) in the composition, and, from the view point of ensuring the stability of the composition, is preferably in a range from 8% by mass to 11% by mass.

As the emulsifier which is Component (C), other emulsifier other than the specific emulsifier may be contained. As the other emulsifier, for example, i) an emulsifier in which the constituent fatty acids are other fatty acid other than oleic acid, or which is a polyglycerin fatty acid ester wherein the degree of polymerization of glycerin is different from the specific emulsifier, or ii) an emulsifier which is an oil-soluble polyglycerin fatty acid ester such as hexaglyceryl polyricinoleate or diglyceryl monostearate may be contained.

Only the specific emulsifier is contained as Component (C) in the fat-and-oil containing composition of the present invention.

The findings of the present inventors, who discovered that when the specific glycerin fatty acid ester that must be included in Component (A) is contained at a high concentration in the fat-and-oil containing composition of the present invention, the specific emulsifier that must be included in Component (C) can be used in combination with glycerin, which is Component (B), are described below.

As an emulsifier which has an aliphatic chain as a hydrophobic group, it is generally known that an emulsifier in which the aliphatic chain is a saturated fatty acid has, because of its lower HLB, a higher solubility in fats and oils compared to an emulsifier in which the aliphatic chain is an unsaturated fatty acid (see, for example, the book "New Edition Surfactant Handbook", edited by Tokiyuki Yoshida et al., published by Kougakutosho Ltd., 1987).

However, according to the findings of the present inventors, it became clear that in a self-emulsification type fat-and-oil containing composition in which a fat-and-oil that contains a high proportion of DHA as the constituent fatty acids thereof is contained at a high concentration, the composition does not exhibits the stability or redispersibility (self-emulsification property) exhibited when using an emulsifier in which the aliphatic chain is an saturated fatty acid, which is generally thought to be effective; and only when a decaglycerin fatty acid ester in which oleic acid having an unsaturated bond in the aliphatic chain is included as the constituent fatty acids is used in combination with glycerin, is it possible to obtain a self-emulsification type fat-and-oil containing composition that has both excellent stability and excellent redispersibility even in a case in which a fat-and-oil that contains a high proportion of DHA as the constituent fatty acids thereof is contained at high concentration.

In addition, the present inventors have discovered that, even in the case of an emulsifier which has oleic acid as an aliphatic chain that is a hydrophobic group, the self-emulsification type fat-and-oil containing composition, in which a fat-and-oil that contains a high proportion of DHA as the constituent fatty acids thereof is contained at a high concentration, does not exhibits stability or redispersibility in the case of polysorbate or sucrose fatty acid ester whose hydrophilic group is sucrose; and only a decaglycerin fatty acid ester which contains oleic acid as the constituent fatty acids thereof (the specific emulsifier) selectively exhibits an excellent effect in emulsification performance.

The content ratio of Component (B) and Component (C) (B:C) is preferably 7:1 to 10:13, more preferably 15:4 to 15:11, on a mass basis.

[(D) Other Component]

The fat-and-oil containing composition of the present invention may contain, as required, other component(s) together with each component of Components (A), (B) and (C). Examples of other component include, for example, other oil components having a physical property and/or a functionality according to the purpose (for example, fats and oils that contain carotenoid pigments such as astaxanthin, lutein and/or lycopene, phospholipids, etc.); flavoring agents; antioxidants; extracts of plants, fruits and so on; and the like.

With respect to oils, an oil(s) may be contained by mixing with Component (A). In this case, the content of the oil(s) is included in the content of Component (A).

The fat-and-oil containing composition of the present invention may be prepared by mixing Components (A), (B) and (C), and an optional component(s).

The fat-and-oil containing composition of the present invention may be prepared, for example, by a method wherein Component (B) and Component (C) are mixed; the resultant is dissolved uniformly in a hot water bath to obtain a mixture; and thereafter, to the mixture, Component (A) is slowly added while stirring well until the total amount thereof is added.

For example, the fat-and-oil containing composition of the present invention may be suitably prepared by a method wherein Component (C) including the specific emulsifier is dissolved in glycerin which is Component (B); and, using as an aqueous phase the resultant and as an oil phase the oil component which is Component (A), the oil phase was added little by little to the aqueous phase to emulsify. To the aqueous phase, additional water may be added.

The fat-and-oil containing composition of the present invention may be suitably applied as a health food, a functional food, a nutritional supplement, or the like.

The fat-and-oil containing composition of the present invention may be suitably prepared as an oral formulation containing the composition. The oral formulation may be provided in a mode in which, without any particular processing, the formulation is directly put into an aqueous liquid such as water to emulsify/disperse, or provided as a capsule formulation.

In addition, the capsule formulation may be either a soft capsule formulation or a hard capsule formulation, and a soft capsule formulation is more preferable from the viewpoints of content leakage and/or ease of intake.

A soft capsule formulation containing the fat-and-oil composition of the present invention may be provided as a soft capsule type formulation in which the fat-and-oil composition is enclosed (loaded) in a soft capsule outer shell.

As the soft capsule outer shell, a soft capsule outer shell is preferable in which the main component is a component derived from an animal raw material such as gelatin or a component derived from a plant raw material such as starch or carrageenan.

When producing a soft capsule formulation, it is desirable from the viewpoint of production applicability that when the fat-and-oil containing composition of the present invention is enclosed (loaded), the temperature of the fat-and-oil containing composition is raised to lower the viscosity or water or the like is added to lower the viscosity. From the viewpoint of the temporal stability of a fat-and-oil containing composition after a soft capsule is completed, it is preferable that water is added to lower the viscosity. Although varying depending on the viscosity of the fat-and-oil containing composition, the amount of water to be added is preferably about 3 to 8% by mass per unit mass of the fat-and-oil containing composition.

Examples of a composition for film formation which is used in forming a soft capsule outer shell include, for example, water-soluble polymers such as starch and carrageenan; plasticizers such as glycerin; water; and compositions for film formation which contain other optional component(s).

A soft capsule formulation may be produced by a known production method such as rotary die method, and, for example, may be produced according to the method as described in JP-A No. 2005-112849.

EXAMPLES

The present invention is described below in detail by way of examples. Unless otherwise specified, "%" is by mass.

[Examples 1 to 14 and Comparative Examples 1 to 15]

<Preparation of Fat-and-Oil Containing Compositions>

The fat-and-oil containing compositions of Examples 1 to 14 and Comparative Examples 1 to 15 were each prepared as described below.

With respect to each of the compositions of Examples 1 to 13 and Comparative Examples 1 to 15, Component (B) and Component (C) of the type and amount shown in Table 1 were mixed, and the resultant was dissolved uniformly in a hot water bath at 80°C to obtain a mixture. Thereafter, to the obtained mixture, Component (A) was slowly added at room temperature (25°C) while stirring well until the total amount thereof was added; whereby each fat-and-oil containing composition was obtained.

With respect to Example 14, the fat-and-oil containing composition was obtained in the same manner as in Example 1, except that Component (B), Component (C) and water of the type and amount shown in Table 1 were mixed to obtain the mixture.

<Evaluation of Fat-and-Oil Containing Compositions>

Using the fat-and-oil containing compositions of Examples 1 to 14 and Comparative Examples 1 to 15, evaluation of the initial stability and the self-emulsifiability was performed.

1. Initial Stability

Each fat-and-oil containing composition was left to stand at 25°C for 1 day in a glass container with a lid. Thereafter, the state of the composition was visually observed, and evaluated in accordance with the following evaluation criteria. The results are also shown in Table 1.

<Evaluation Criteria>

   A: no separation is found, the composition is uniform, and stability is excellent
   B: the oil component is not separated from the composition, and the composition is stable
   C: the oil component is separated, and is seeping out to the surface of the composition
   D: the oil component is completely separated from the composition

The oil component herein refers to an oil component(s) such as Component (A).

2. Self-Emulsification Properties

For the fat-and-oil containing compositions in which the evaluation result of the initial stability was "A" or "B", 50 ml of water at 25°C was added to 100 mg of each fat-and-oil containing composition; the resultant was stirred with a magnetic stirrer and dispersed; and the self-emulsification properties when contacted with water was visually observed, and evaluated in accordance with the following evaluation criteria. The results are also shown in Table 1.

   A: self-emulsification occurred to a fine degree
   B: self-emulsification occurred without oil droplet coalescence
   C: self-emulsification occurred, but oil droplet coalescence was observed or turbidity was high (dispersion diameter was large)
   D: no self-emulsification occurs

[Table 1]

| | | | Examples | | | | | | | | | | | | | | Comparative Examples | | | | | | | | | | | | | |
|---|---|---|---|---|---|---|---|---|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 1 | 2 | 3 | 4 | 5 | 6 | 7 | 8 | 9 | 10 | 12 | 13 | 14 | 15 |
| Composition | Component (A) (Oil component) | Fat-and-Oil A (g): the specific glycerin fatty acid ester | 30 | 50 | 50 | 70 | | 90 | 70 | 70 | 70 | 70 | 70 | 70 | 70 | 70 | 25 | 50 | 70 | 90 | 110 | 70 | 70 | 70 | | | 70 | 70 | 70 | 70 |
| | | Fat-and-Oil B (g): the specific glycerin fatty acid ester | | | | | 70 | | | | | | | | | | | | | | | | | | | | | | | |
| | | Fat-and-Oil C (g) | | | | | | | | | | | | | | | | | | | | | | | 70 | | | | | |
| | | Fat-and-Oil D (g) | 40 | 20 | | | | | | | | | | | | | 45 | | | | | | | 70 | | | | | | |
| | Component (B) | Glycerin (g) | 20 | 20 | 20 | 20 | 20 | 20 | 10 | 15 | 25 | 20 | 20 | 30 | 40 | 20 | 20 | 20 | 20 | 20 | 20 | 20 | 20 | 20 | 20 | 20 | 20 | 13 | 10 | 60 |
| | Component (C) (Emulsifier) | Emulsifier A (g) | | | | | | | | | | | | | | | | 10 | 10 | 10 | | | | | | | | | | |
| | | Emulsifier B (g): the specific emulsifier | 10 | 10 | 10 | 10 | 10 | 10 | 10 | 10 | 10 | 5 | 13 | 10 | 10 | 10 | 10 | | | | 10 | | | | 10 | 10 | 3 | 17 | 5 | 10 |
| | | Emulsifier C (g) | | | | | | | | | | | | | | | | | | | 10 | | | | | | | | | |
| | | Emulsifier D (g) | | | | | | | | | | | | | | | | | | | | 10 | | | | | | | | |
| | | Emulsifier E (g) | | | | | | | | | | | | | | | | | | | | | 10 | | | | | | | |
| | Other | Water (g) | | | | | | | | | | | | | 5 | | | | | | | | | | | | | | | |
| Content Ratio | | Component (A): the ratio (% by mass) of the oil component | 70 | 70 | 62.5 | 70 | 70 | 75 | 77.8 | 73.7 | 66.7 | 73.7 | 68 | 63.6 | 58.3 | 66.7 | 70 | 62.5 | 70 | 75 | 78.6 | 70 | 70 | 70 | 70 | 70 | 75.3 | 70 | 82.4 | 50 |
| | | Component (B): the ratio (% by mass) of the glycerin | 20 | 20 | 25 | 20 | 20 | 16.7 | 11.1 | 15.8 | 23.8 | 21.1 | 19.4 | 27.3 | 33.3 | 19 | 20 | 25 | 20 | 16.7 | 14.3 | 20 | 20 | 20 | 20 | 20 | 21.5 | 13 | 11.8 | 43 |
| | | Component (C): the ratio (% by mass) of the emulsifier | 10 | 10 | 12.5 | 10 | 10 | 8.3 | 11.1 | 10.5 | 9.5 | 5.3 | 12.6 | 9.1 | 8.3 | 9.5 | 10 | 12.5 | 10 | 8.3 | 7.1 | 10 | 10 | 10 | 10 | 10 | 3.2 | 17 | 5.9 | 7.1 |
| | | The ratio of DHA in the constituent fatty acids contained in the oil component | 30 | 50 | 71 | 70 | 73 | 73 | 70 | 72 | 71 | 70 | 71 | 70 | 72 | 70 | 25 | 70 | 71 | 70 | 72 | 71 | 70 | 71 | 15 | 0 | 71 | 73 | 72 | 71 |
| Evaluation | | Initial Stability | A | A | A | A | A | B | B | B | A | A | B | A | A | A | B | B | B | B | D | D | D | D | D | D | B | D | C | B |
| | | Self-emulsification property | B | B | A | A | A | B | B | B | A | B | B | A | A | A | C | C | C | C-D | - | | - | - | - | - | C | - | C | C |

* The content ratios of Components (A) to (C) represent the content ratios (% by mass) of each component relative to the total mass of Components (A), (B) and (C).

Details of the individual components described in Table 1 are as follow.

<Component (A): Oil component>

- Fat-and-Oil A: a fat-and-oil containing raw material in which not less than 70% by mass of the constituent fatty acids are docosahexaenoic acid, and the average number of fatty acid esterification is about 2.7 or more (trade name: ALGATRIUM (manufactured by Brudy Technology)) (the specific glycerin fatty acid ester)
- Fat-and-Oil B: a fat-and-oil containing raw material in which 46% by mass of the contained fatty acids are docosahexaenoic acid, and the average number of fatty acid esterification is about 2.8 or more (trade name: DHA-46A, manufactured by Maruha Nichiro Foods, inc.) (the specific glycerin fatty acid ester)
- Fat-and-Oil C: a fat-and-oil containing raw material in which 22% by mass of the contained fatty acids are docosahexaenoic acid, and the average number of fatty acid esterification is about 2.8 or more (trade name: DHA-22, manufactured by Maruha Nichiro Foods, inc.)
- Fat-and-Oil D: a fat-and-oil containing raw material which is triglyceride in which the fatty acids are caprylic acid (saturated fatty acid C8: docosahexaenoic acid is not contained), and the average number of fatty acid esterification is about 2.9 or more (trade name: COCONARD RK, manufactured by Kao Corporation)

<Component (B)>

- Glycerin (GLYCERIN (FOOD ADDITIVE), manufactured by Kao Corporation) (Component (B))

<Component (C): Emulsifier>

- Emulsifier A: a decaglycerin fatty acid ester in which the aliphatic chains are stearic acid ($C_{18}$ saturated fatty acid) (trade name: SUNSOFT Q-18S, manufactured by Taiyo Kagaku Co., Ltd.)
- Emulsifier B: a decaglycerin fatty acid ester in which the aliphatic chains are oleic acid ($C_{18}$ unsaturated fatty acid) (trade name: POEM J-0381V, manufactured by Riken Vitamin Co., Ltd.) (the specific emulsifier)
- Emulsifier C: a decaglycerin fatty acid ester in which the aliphatic chains are lauric acid (C12 saturated fatty acid) (trade name: SUNSOFT Q-12S, manufactured by Taiyo Kagaku Co., Ltd.)
- Emulsifier D: polysorbate 80 (trade name: WILSURF TF-80, NOF CORPORATION)
- Emulsifier E: a sucrose fatty acid ester in which the aliphatic chains are oleic acid (trade name: Q-1570Y, manufactured by Mitsubishi-Kagaku Foods Corporation)

As shown in Table 1, it is found that the fat-and-oil containing compositions of Examples are self-emulsification type compositions which have high initial stability and good redispersibility.
Each fat-and-oil containing composition obtained in Examples is preferred to be enclosed in a soft capsule outer shell to form a soft capsule formulation.

## Claims

1. A fat-and-oil containing composition which comprises at least (A) an oil component, (B) glycerin, and (C) an emulsifier, the composition comprising:

   a glycerin fatty acid ester, in which from 30 % by mass to 75 % by mass of constituent fatty acids are docosahexaenoic acid and is a glycerin fatty acid ester in which an average number of fatty acid esterification of hydroxy groups in the glycerin skeleton is from 2.5 to 3.0, as (A) the oil component; and
   a decaglycerin fatty acid ester, in which constituent fatty acids are oleic acid, as (C) the emulsifier,
   wherein respective content ratios of (A) the oil component, (B) the glycerin and (C) the emulsifier are within the following ranges relative to the total mass of (A) the oil component, (B) the glycerin and (C) the emulsifier:

   (A) the oil component: from 55 % by mass to 78 % by mass,

(B) the glycerin: from 10 % by mass to 35 % by mass, and

(C) the emulsifier: from 5 % by mass to 13 % by mass, and

wherein only the specific emulsifier decaglycerin fatty acid ester, in which constituent fatty acids are oleic acid, is contained as (C) the emulsifier.

**2.** An oral formulation comprising the fat-and-oil containing composition of claim 1.

**Patentansprüche**

**1.** Fett- und ölhaltige Zusammensetzung, die zumindest (A) eine Ölkomponente, (B) Glycerin und (C) einen Emulgator umfasst, wobei die Zusammensetzung umfasst:

als Ölkomponente (A) einen Glycerin-Fettsäureester, worin 30 Masse-% bis 75 Masse-% der aufbauenden Fettsäuren aus Docosahexaensäure bestehen und der ein Glycerin-Fettsäureester ist, worin die mittlere Fettsäureveresterungszahl der Hydroxygruppen in dem Gleceringerüst von 2,5 bis 3,0 beträgt,; und

als Emulgator (C) einen Decaglycerin-Fettsäureester, worin die aufbauenden Fettsäuren aus Ölsäure bestehen, ,

worin die entsprechenden Mengenverhältnisse der Ölkomponente (A), des Glycerins (B) und des Emulgators (C) innerhalb der folgenden Bereiche liegen, relativ zur Gesamtmasse der Ölkomponente (A), des Glycerins (B) und des Emulgators (C):

(A) die Ölkomponente: von 55 Masse-% bis 78 Masse-%,

(B) das Glycerin: von 10 Masse-% bis 35 Masse-% und

(C) der Emulgator: von 5 Masse-% bis 13 Masse-%, und

worin als Emulgator (C) nur der spezifische Emulgator Decaglycerin-Fettsäureester, worin die aufbauenden Fettsäuren aus Ölsäure bestehen, enthalten ist.

**2.** Orale Formulierung, umfassend die fett- und öl-haltige Zusammensetzung gemäß Anspruch 1.

**Revendications**

**1.** Composition contenant graisse et huile qui comprend au moins (A) un composant huileux, (B) de la glycérine, et (C) un émulsifiant, la composition comprenant :

un ester d'acide gras de glycérine, dans lequel de 30 % en masse à 75 % en masse d'acides gras constitutifs sont de l'acide docosahexaénoïque et est un ester d'acide gras de glycérine dans lequel un nombre moyen d'estérification de groupes hydroxy par les acides gras dans le squelette de la glycérine est de 2,5 à 3,0, en tant que (A) le composant huileux ; et

un ester d'acide gras de décaglycérine dans lequel les acides gras constitutifs sont de l'acide oléique, en tant que (C) l'émulsifiant,

dans laquelle les taux de teneur respectifs de (A) le composant huileux, (B) la glycérine et (C) l'émulsifiant se trouvent dans les plages suivantes relativement à la masse totale de (A) le composant huileux, (B) la glycérine et (C) l'émulsifiant :

(A) le composant huileux : de 55 % en masse à 78 % en masse,

(B) la glycérine : de 10 % en masse à 35 % en masse, et

(C) l'émulsifiant : de 5 % en masse à 13 % en masse, et

dans laquelle seul l'émulsifiant d'ester d'acide de décaglycérine, dans lequel les acides gras constitutifs sont de l'acide oléique, est contenu en tant que (C) l'émulsifiant.

**2.** Formulation orale comprenant la composition contenant graisse et huile selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009114157 A

- JP 2005112849 A

**Non-patent literature cited in the description**

- Standard Methods for Testing the Physical Properties of Fats and Oils

- New Edition Surfactant Handbook. Kougakutosho Ltd, 1987